# EUROPEAN PATENT APPLICATION

(11) **EP 1 576 977 A1**
(43) Date of publication of application: **21.09.2005**
(21) Application number: 04004160.0
(22) Date of filing: 25.02.2004
(51) Int. Cl.: A61M 5/32

(54) **Syringe with retractable needle**

(30) Priority: 18.04.2003 US 418062
(71) Applicant: Life-Shield Products, Inc., Taipei (TW)
(72) Inventor: Shih, Chao-Hua, Wugu Shiang Taipei (TW)
(74) Representative: Meyer, Ludgerus

(57) **Abstract**

A safety syringe comprises a needle set (2), a barrel (1) and a plunger (3). Wherein the front end of the needle set (21) is connected with a needle (22), while the rear end is provided with a first coupling portion (212), the bottom of the needle set has on the periphery thereof at least an annular flange (211). The barrel is hollow, its front end is formed as a socket to connect the needle set; the socket has on its inner wall two mutually neighboring annular flanges (111,112). The rear end of the barrel is used for slipping in the plunger of which the front end has a piston (32); a second coupling portion (33) is provided on the front end of the piston. Hence when the needle set is connected to the needle-set socket (11), the said at least an annular flange on the periphery of the needle set is located between the two annular flanges on the inner wall of the needle-set socket to increase the tightness of connection. When the front end of the plunger is pushed to the topmost position, the first (212) and second (33) coupling portions mutually connect, so that when the plunger is pulled back, the needle set can be pulled back into the barrel.

## Description

### BACKGROUND OF THE INVENTION

### 1. Field of the invention

The present invention is related to a safety retractable type syringe; and especially to an improved engaging structure of the barrel and retractable needle set, easy for manufacturing and use, which can be applied to standard needle syringes as well as general retractable syringes.

### 2. Description of the Prior Art

The U.S. Patent No. 5,931,813, owned by the same patentee as the present invention, disclosed a safety retractable type syringe structure, the structure generally is provided beneath a needle seat C1 of a retractable type needle set C (including the needle seat C1 and a needle C2) with a first specific coupling portion C11, and is provided on the upper end of a plunger B with a second coupling member B1 connectable with the first coupling portion C11. After injection, the plunger B received in a barrel A is pushed upwardly to its topmost position to make connecting of the first coupling portion C11 with the second coupling member B1, thereby, the retractable type needle set C engaged on the upper end of the inner wall of the barrel A is pulled back into the barrel A ready for discarding.

However, the connecting or engaging structure for the retractable type needle set C and the barrel A is very important because of the problem of tolerance in manufacturing. Sometimes the needle set C is hard to be inserted into and connected with the barrel A; sometimes it is inserted into the front end of the barrel A too loosely, and because it only relies on the engagement of a flange C 12 provided on the needle seat C1 of the retractable type needle set C with the front end of the inner wall of the barrel A, the engagement is not firm. In view of this, the inventor of the present invention had the motive to try to make improvement and to develop an improved engaging structure of a retractable type needle set and a barrel.

### SUMMARY OF THE INVENTION

The primary object of the present invention is to provide an improved engaging structure of the retractable type needle set and barrel, easy for manufacturing and use, and in which the liquid medicine is well sealed.

The secondary object of the present invention is to provide a retractable safety syringe capable of using a standard needle set as well as a not-standard needle set.

To achieve the above objects, the present invention is comprised of a needle set, a barrel and a plunger. Wherein the front end of the needle set is connected with a needle, while the rear end is provided with a first coupling portion, the bottom of the needle set is provided on the periphery thereof at least with an annular flange; the barrel is a hollow pipe, its front end is formed as a socket for connecting thereto of the needle set, the socket is provided on the inner wall thereof with two mutually neighboring annular flanges (the lower annular flange can be substituted with at least one protrusion). The rear end of the barrel is used for slipping in the plunger of which the front end is provided with a piston, a second coupling portion is provided on the front end of the piston. Thereby, when the needle set is connected into the needle-set socket and engaged therein, the aforesaid at least an annular flange provided on the periphery of the needle set is located between the two annular flanges provided on the inner wall of the needle-set socket, and thus overcome the problem of tolerance in manufacturing to make easier for manufacturing, assembling and use as well as increasing the effect of sealing liquid medicine. When the front end of the plunger is pushed to the topmost position, the first coupling portion and the second coupling portion connect with each other, so that when the plunger is pulled back, the needle set can be pulled back into the barrel.

The present invention will be apparent in its features after reading the detailed description of the preferred embodiments thereof in reference to the accompanying drawings.

### BRIEF DESCRIPTION OF THE DRAWINGS

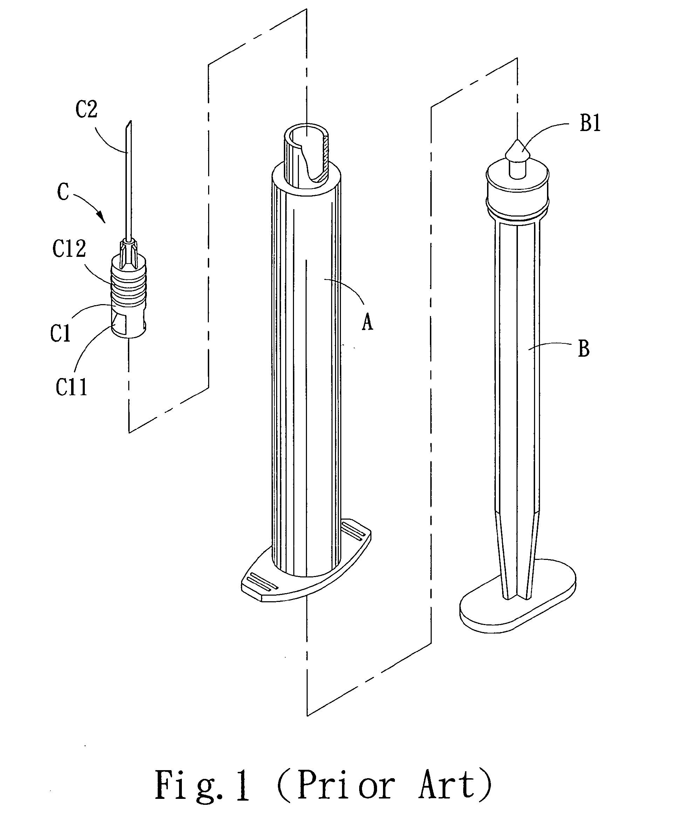

Fig. 1 is an analytic perspective view showing the elements of a conventional syringe;
Fig. 2 is an analytic perspective view showing the first embodiment of the present invention;
Fig. 3 is a sectional view showing assembling of the elements of the first embodiment of the present invention;
Fig. 3a is a partially exploded perspective view showing the upper end portion of the second embodiment of the present invention;
Fig. 4 is a sectional view showing assembling of the third embodiment of the present invention;
Fig. 4a is an enlarged sectional view from Fig. 4.

### DETAILED DESCRIPTION OF THE PREFERRED EMBODIMENT

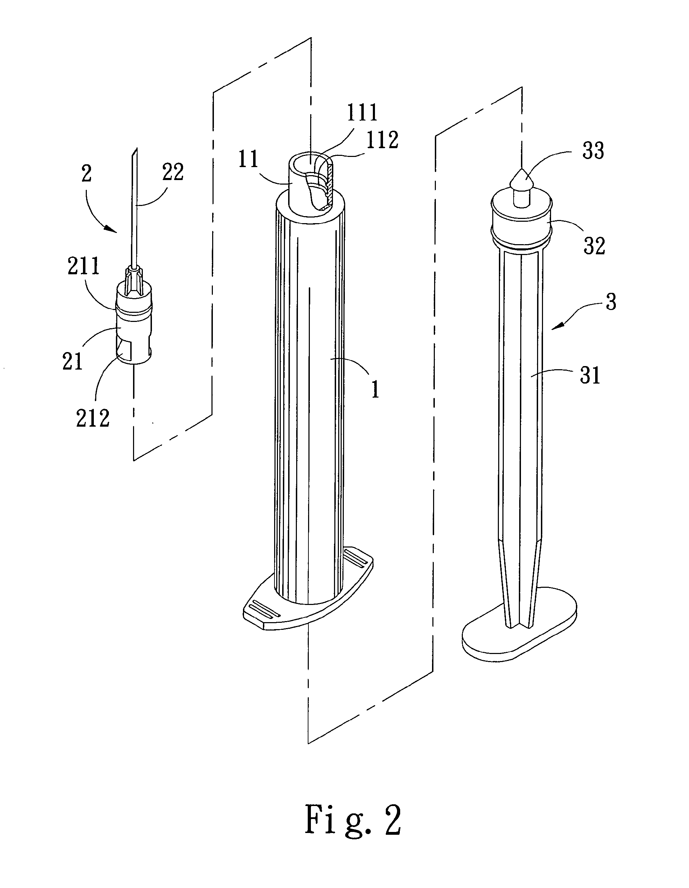

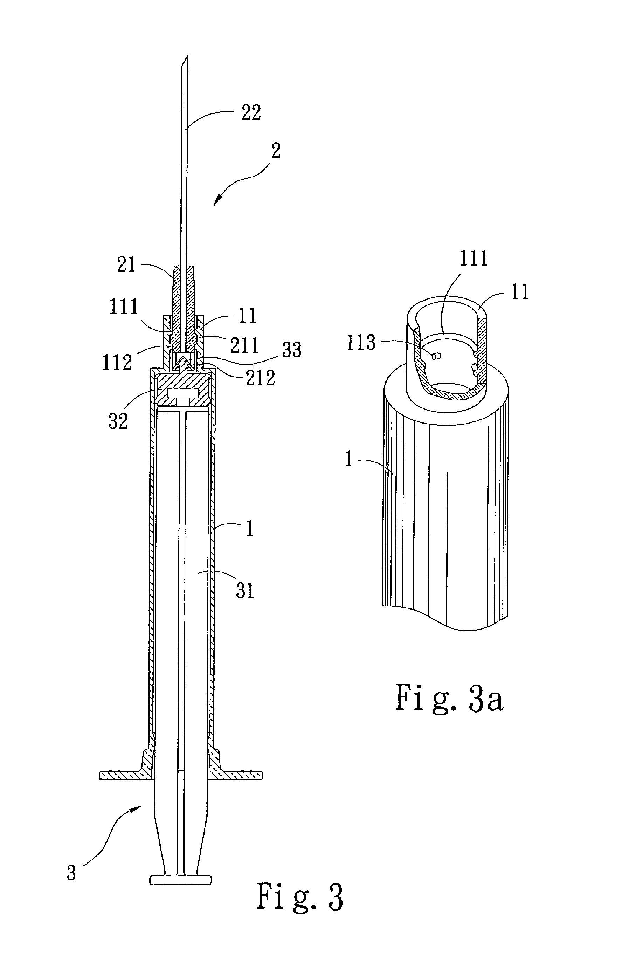

Referring to Fig. 2 showing a first embodiment of the present invention, the embodiment comprises a barrel 1, a retractable type needle set 2 and a plunger 3. Wherein the barrel 1 is a hollow pipe, its front end is reduced to form a needle-set socket 11, the needle-set socket 11 is provided on the inner wall thereof with two annular flanges: an upper flange 111 and a lower flange 112; and as shown in Fig. 3a, the lower annular flange 112 originally provided on the inner wall of the needle-set socket 11 can alternatively be at least one protrusion 113 provided on a horizontal circle.

The retractable type needle set 2 includes a needle seat 21 and a needle 22, the rear end of the needle seat 21 is provided with a first coupling portion 212; the external diameter of the needle seat 21 is coincident with the inner diameter of the needle-set socket 11, so that the needle seat 21 can be engaged with the latter; the external wall of the middle section of the needle seat 21 is provided with at least an annular flange 211 to increase the engaging function in connecting and sealing function that the liquid medicine will not leak out. In this embodiment, the first coupling portion 212 is a coupling portion with a recess, while the recess is formed from more than one detent piece.

The plunger 3 is generally a pusher rod 31, the front end thereof has a piston 32; a second coupling portion 33 is provided on the front end of the piston 32. In this embodiment, the second coupling portion 33 is a protruding hook portion. The plunger 3 is provided for slipping into the barrel 1 to move to and fro therein.

Referring to Figs. 3 and 3a, the retractable type needle set 2 is inserted into the needle-set socket 11 of the barrel 1, and the flange 211 of the needle seat 21 is located and engaged between the upper flange 111 of the needle-set socket 11 and the lower flange 112 (or the at least one protrusion 113), so that the retractable type needle set 2 can be more tightly connected with the needle-set socket 11. When the retractable type safety syringe is used, it shall be sealed during the processes of medicine drawing out and injection; while after injection, the plunger 3 is pushed to its topmost position, the second coupling portion 33 provided on the front end of the piston 32 can be connected with the first coupling portion 212 of the needle seat 21 and the protruding hook portion of the second coupling portion 33 is connected with the ends of the detent pieces. At this time, the plunger 3 is pulled back, then the retractable type needle set 2 including the needle 22 can be drawn back into barrel 1.

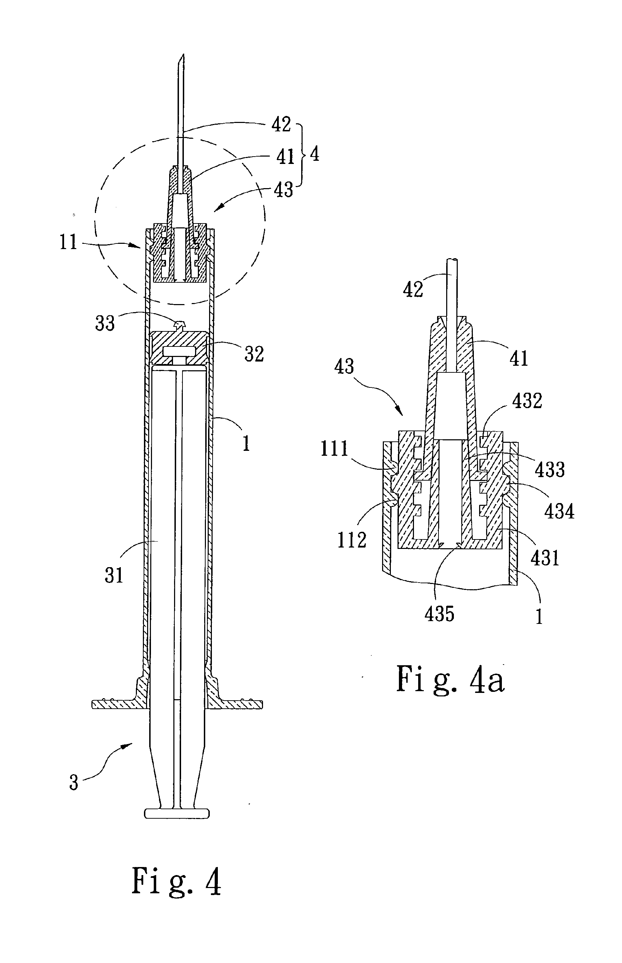

Referring to Figs. 4 and 4a, the third embodiment of the present invention is comprised of a barrel 1, a standard needle set 4 and a plunger 3. Wherein the barrel 1 is a hollow pipe, its front end is formed as a needle-set socket 11; the needle-set socket 11 is provided on the inner wall thereof with two annular flanges: an upper flange 111 and a lower flange 112 (the lower annular flange 112 can be at least a protrusion provided on a horizontal circle).

As shown in Fig. 4, the standard needle set 4 includes a needle seat 41, a standard needle 42 and a bottom seat (namely, an adapter) 43. The needle 42 is connected on the front end of the needle seat 41 which can be detached from and connected to the bottom seat 43 of the needle set 4; the bottom seat 43 is provided with an annular outer hub 431 and an inner hub 433. The inner wall of the outer hub 431 has a thread 432 for screw connecting the needle seat 41, while external wall of the outer hub 431 has at least an annular flange 434. The rear end of the inner hub 433 has a first coupling portion 435; in this embodiment, the needle seat 41 and the bottom seat 43 are rotating connected as a Lure lock, while in practicing, it can alternatively be engaged directly with the standard needle set 4 as a Lure slip structure.

The standard needle set 4 is inserted into the needle-set socket 11 formed on the front end of the barrel 1. The flange 434 of the bottom seat 43 is engaged between the upper flange 111 of the needle-set socket 11 and the lower flange 112, so that the syringe will be well sealed during the processes of medicine drawing out and injection. When the front end of the plunger 3 is pushed to the topmost position after injection, the second coupling portion 33 on the front end of the piston 32 is connected with the first coupling portion 435 of the bottom seat 43 to pull the plunger 3 rearwardly, and the standard needle set 4 can then be pulled into the barrel 1.

The above stated in referring to the drawings are preferred embodiments of the present invention, for example, wherein the first coupling portion 212 of the needle seat 21 on the retractable type needle set 2 and the first coupling portion 435 of the bottom seat 43 of the standard needle set 4 are both provided with recesses; while the second coupling portion 33 on the plunger 3 is provided with a protruding hook portion. However, in practicing, other shapes and structures able to get the function of coupling can alternatively be adopted. By all means, the first coupling portion 212, 435 respectively of the needle seat 21 of the retractable type needle set 2 and the bottom seat 43 can be embodied as hook portions, while the second coupling portion 33 on the plunger 3 can be embodied as a recess, and the same function of coupling or connection can likely be achieved.

Accordingly, the present invention has the following advantages:
1. By providing the two annular flanges on the inner wall of the needle-set socket:
   an upper flange and a lower flange (or at least one protrusion), the flanges on the needle seat of the needle set can be engaged therewith; this can overcome the problem of tolerance in manufacturing to make easier for manufacturing the components.
2. Overcoming of the problem of tolerance in manufacturing the components not only makes easier for manufacturing, but also makes quite convenient for assembling and use.
3. The syringe can be well sealed during the processes of medicine drawing out and injection.

As stated in the above disclosed, the present invention can surely attain its expected objects to provide an improved engaging structure of a retractable needle set and a barrel, the structure undoubtedly has its practical value of utility.

It will be apparent to those skilled in this art that various modifications or changes made to the elements of the present invention without departing from the spirit of this invention shall fall within the scope of the appended claims.

## Claims

1. A safety retractable type syringe comprising:
a needle set, the front end of said needle set being connected with a needle, while the rear end of said needle set being provided with a first coupling portion;
a barrel, being a hollow pipe, the front end thereof being formed as a needle-set socket provided for connecting and engaging said needle set therein;
a plunger in the form of a pusher rod, the front end thereof having a piston, said plunger being provided for slipping into said barrel to move to and fro therein for drawing out or injecting liquid medicine; a second coupling portion being provided on the front end of said piston which can be connected with said first coupling portion; when the front end of said plunger being pushed to a topmost position, said first coupling portion and said second coupling portion connecting with each other, so that when said plunger being pulled back, said needle set being pulled back into said barrel;
**characterized in that** said needle-set socket is provided on the inner wall thereof with two mutually neighboring annular flanges; the bottom of said needle set is provided on the periphery thereof at least with an annular flange, so that when said needle set is connected with said needle-set socket, said at least an annular flange provided on said periphery of said bottom of said needle set is located between said two annular flanges provided on said inner wall of said needle-set socket.

2. The safety retractable type syringe as claimed in claim 1, wherein said needle set is a retractable type needle set comprising a needle and a needle seat, said needle is fixed on the front end of said needle seat, said needle seat is a bottom seat of said needle set, and said front end of said barrel is reduced to form said needle-set socket.

3. The safety retractable type syringe as claimed in claim 1, wherein said needle set includes a needle, a needle seat and a bottom seat (namely, an adapter), said needle is connected on the front end of said needle seat which is adapted to detaching from and connecting to said bottom seat of said needle set.

4. A safety retractable type syringe comprising:
a needle set, the front end of said needle set being connected with a needle, while the rear end of said needle set being provided with a first coupling portion;
a barrel, being a hollow pipe, the front end thereof being formed as a needle-set socket provided for connecting said needle set therein;
a plunger in the form of a pusher rod, the front end thereof having a piston, said plunger being provided for slipping into said barrel to move to and fro therein for drawing out or injecting liquid medicine; a second coupling portion being provided on the front end of said piston which ca be connected with said first coupling portion; when the front end of said plunger being pushed to a topmost position, said first coupling portion and said second coupling portion connecting with each other, so that when said plunger being pulled back, said needle set being pulled back into said barrel;
**characterized in that** said needle-set socket is provided on the inner wall thereof with an annular flange, at least one protrusion provided below said annular flange; the bottom of said needle set is provided on the periphery thereof at least with an annular flange, so that when said needle set is connected with said needle-set socket, said at least an annular flange provided on said periphery of said bottom of said needle set is located between said annular flange provided on said inner wall of said needle-set socket and said at least one protrusion.

5. The safety retractable type syringe as claimed in claim 4, wherein said needle set is a retractable type needle set comprising a needle and a needle seat, said needle is fixed on the front end of said needle seat, said needle seat is a bottom seat of said needle set, and said front end of said barrel is reduced to form said needle-set socket.

6. The safety retractable type syringe as claimed in claim 4, wherein said needle set includes a needle, a needle seat and a bottom seat (namely an adapter), said needle is connected on the front end of said needle seat which is adapted to detaching from and connecting to said bottom seat of said needle set.
